# EUROPEAN PATENT APPLICATION

(11) **EP 4 578 446 A1**
(43) Date of publication of application: **02.07.2025**
(21) Application number: 24219708.5
(22) Date of filing: 13.12.2024
(51) Int. Cl.: A61K 31/133, A61K 31/196, A61K 31/198, A23K 50/50, A61K 36/31, A61K 36/55, A61P 15/08

(54) **COMPOSITION FOR USE IN THE TREATMENT OF OVULATORY SUB- OR INFERTILITY IN EQUIDAE**

(30) Priority: 15.12.2023 BE 202306020
(71) Applicant: Curafyt BV, 9320 Aalst (BE)
(72) Inventor: DE CLERCK, Valérie, 9320 Aalst (BE)
(74) Representative: Brantsandpatents bv

(57) **Abstract**

The present invention relates to a composition for use in treating ovulatory infertility in equids, the composition comprising a mixture of one or more methyl donors. The invention also relates to a method for collecting one or more unfertilized oocytes from the follicles of the ovaries of a subject of the equid group and *ex vivo* fertilizing these one or more oocytes, wherein prior to collecting the one or more unfertilized oocytes a compound is administered to the subject. The invention also relates to a feed additive for equids.

## Description

### TECHNICAL FIELD

The invention relates to a composition and a feed additive for use in the treatment of fertility problems, more specifically in the treatment of ovulatory sub- or infertility in equids, such as horses and ponies. The invention also relates to a method for collecting one or more unfertilized oocytes from the follicles of the ovaries of a mare and ex *vivo* fertilizing these one or more oocytes, wherein prior to collecting the one or more unfertilized oocytes a compound is administered to the subject, wherein the composition optimizes ovulatory fertility.

### PRIOR ART

The reproductive performance of mares is lower than that of other domesticated animals in terms of fertilization rate.

There are various factors that can contribute to fertility problems in mares, for example endometritis, anovulatory follicles, the age of the mares, and/or the presence of metabolic syndrome. Furthermore, oocyte maturation is also an important factor, both when fertilization takes place *in vivo* and ex *vivo.*

The maturation of oocytes is a complicated and vital process that is used to achieve the full competence required by both the oocyte itself and the early embryonic development of a fertilized oocyte. In humans and animals, multiple factors, including epigenetic molecules and various signaling pathways, have been identified and proven to be crucial for this maturation. They not only regulate the maturation of oocytes but also coordinate with each other to ensure good quality of the oocytes, both of which are necessary to guarantee optimal fertility.

Methylation, the process by which methyl groups are added to DNA and histones, plays a crucial role in the epigenetic regulation of genes. Disrupted methylation can have significant consequences for the quality of oocytes and the development of embryos in horses, as well as in other mammals.

Dietary supplements for horses are known. For example, WO1996032850 and US5595982 describe the use of certain methyl donors in dietary supplements. Often, the known dietary supplements are inadequate, and fertility problems persist.

The present invention aims to find a solution for at least some of the above problems.

### SUMMARY OF THE INVENTION

The invention relates to a composition for use in treating ovulatory sub- or infertility in equids. More specifically, the invention relates to a composition according to claim 1, the composition comprising a mixture of one or more methyl donors.

Further embodiments of the composition are described in claims 2-10. In a preferred embodiment, the composition further comprises glucobrassicin or a source of glucobrassicin. In a further or alternative preferred embodiment, the composition further comprises sulforaphane and/or one or more plant-based components as a source of sulforaphane. In a further or alternative preferred embodiment, the composition further comprises lignans or a source of lignans. In a further or alternative preferred embodiment, the composition further comprises cysteine.

More specifically, it has been found that the composition has an optimal effect on methylation processes involved in gene expression and cell metabolism, including gene transcription of genes involved in oocyte maturation, an important process with a significant impact on the fertility of the animal. The current invention is capable of treating ovulatory sub- or infertility by optimizing the process of oocyte maturation to increase the number of oocytes and/or the quality of the oocytes. The current invention makes it possible, for example, to increase the number and/or quality of the unfertilized oocytes that can be collected directly from the follicles of a mare's ovaries via OPU. In addition to treating ovulatory disorders, the current invention also makes it possible to optimize the OPU procedure and subsequent fertilization due to the increased number of oocytes and the improved quality of the oocytes. This makes it possible to produce offspring from valuable or genetically important mares that would otherwise be unable to breed successfully due to age, health, or other complications.

In a second aspect, the invention therefore also relates to a method for collecting one or more unfertilized oocytes from the follicles of the ovaries of a subject of the equid group. More specifically, the invention relates to a method according to claim 11, wherein prior to collecting the one or more unfertilized oocytes, a composition is administered to the subject, the composition comprising a mixture of one or more methyl donors.

In a further aspect, the invention relates to a method for ex *vivo* fertilizing one or more oocytes of a subject of the equid group. More specifically, the invention relates to a method according to claim 12, comprising:
- collecting one or more unfertilized oocytes from the follicles of the ovaries of the subject;
- performing ICSI (Intracytoplasmic Sperm Injection) and/or IVF (*in vitro* fertilization) for fertilizing the one or more collected oocytes, wherein prior to collecting the one or more unfertilized oocytes, a composition is administered to the subject, the composition comprising a mixture of one or more methyl donors.

In a final aspect, the invention relates to a feed additive for equids. More specifically, the invention relates to a feed additive according to claim 14, comprising:
- Between 15 and 25 weight percent broccoli extract;
- Between 5 and 23 weight percent linseed extract;
- Between 10 and 15 weight percent cysteine;
- Between 5 and 15 weight percent choline or choline derivative;
- Between 8 and 12 weight percent betaine.

### DETAILED DESCRIPTION

The invention relates to a composition and a feed additive for use in treating sub- or infertility in equids, more specifically for treating ovulatory infertility. The invention also relates to a method for collecting one or more unfertilized oocytes directly from the follicles of the ovaries of a subject of the equid group and subsequently fertilizing these one or more collected oocytes, wherein prior to collecting the one or more oocytes, a composition is administered to the subject.

The current invention is capable of treating ovulatory sub- or infertility by optimizing the process of oocyte maturation to increase the number of oocytes and/or the quality of the oocytes. The current invention makes it possible, for example, to increase the number and/or quality of the unfertilized oocytes that are collected directly from the follicles of a mare's ovaries through OPU. In addition to treating ovulatory disorders that play a role in *in vivo* fertilization, the current invention also makes it possible to optimize the OPU procedure and subsequent ex *vivo* fertilization due to the increased number of oocytes and/or the improved quality of the oocytes that can be collected. Optimization of oocyte quality increases the chances of successful maturation of the oocytes and successful embryonic development, particularly just after fertilization. As a result, the current invention is also capable of increasing the percentage of fertilized oocytes that develop further into a blastocyst. This makes it possible to produce offspring from valuable or genetically important mares that would otherwise be unable to breed successfully due to age, health, a sports career, or other complications.

Unless otherwise defined, all terms used in the description of the invention, including technical and scientific terms, have the meaning as commonly understood by a person skilled in the art to which the invention pertains. For a better understanding of the description of the invention, the following terms are explained explicitly.

In this document, "a" and "the" refer to both the singular and the plural, unless the context presupposes otherwise. For example, "a segment" means one or more segments.

When the term "around" or "about" is used in this document with a measurable quantity, a parameter, a duration or moment, and the like, then variations are meant of approx. 20% or less, preferably approx. 10% or less, more preferably approx. 5% or less, even more preferably approx. 1% or less, and even more preferably approx. 0.1% or less than and of the quoted value, insofar as such variations are applicable in the described invention. However, it must be understood that the value of a quantity used where the term "about" or "around" is used, is itself specifically disclosed.

The terms "comprise," "comprising," "consist of," "consisting of," "provided with," "have," "having," "include," "including," "contain," "containing" are synonyms and are inclusive or open terms that indicate the presence of what follows, and which do not exclude or prevent the presence of other components, characteristics, elements, members, steps, as known from or disclosed in the prior art.

"Oocyte maturation" as used herein, encompasses three developmental programs essential for the production of an oocyte suitable for fertilization and embryogenesis:
(1) nuclear maturation, which comprises the resumption and completion of the first meiotic division and the maintenance of a stable metaphase-II arrest, (2) epigenetic maturation, which occurs during oocyte growth and results in genomic modifications that regulate gene expression, and (3) cytoplasmic maturation, commonly defined as the processes in the cytoplasm of developing oocytes that are essential for fertilization and early embryonic development.

The term "folliculogenesis" as used herein, encompasses the growth of a follicle, from the moment it is released from the reserve of follicles established during the embryonic period, to the moment it either ovulates or becomes atretic. It is a continuous process, whereby follicles are released from the reserve daily.

"Follicle" as used herein, comprises round to ovoid structures whose size depends on the growth stage and animal species and contains the future ovum or oocyte. During the embryonic phase, germ cells migrate to the genital ridge, settle between the surface epithelium, multiply, and thus form the germinal epithelium. During embryonic gametogenesis, female germ cells (oogonia) evolve into oocytes (ovocytes) I, start the 1st meiosis, but stop in the prophase. These oocytes I grow into the cortical tissue and form primordial follicles there. When the oocyte I ovulates, the 1st meiosis will occur, resulting in an oocyte II and a polar body I. The oocyte II proceeds to the 2nd meiotic division as soon as fertilization occurs, after which the polar bodies are resorbed.

"Ovulatory sub- or infertility" as used herein, is defined as sub- or infertility due to defective oocyte maturation and/or folliculogenesis and/or ovulation. When one or more factors during oocyte maturation, folliculogenesis, and/or ovulation are disrupted, ovulatory infertility can occur, for example, as a result of a poor response of the ovaries to stimulation, stagnation of oocyte maturation, poor oocyte quality, and failed fertilization or early embryonic developmental arrest due to, for example, poor oocyte quality. "Infertility" as used here, is the inability to reproduce. Infertility is defined as the established inability of an individual to have children. Until this is determined, the term "subfertility" is used, indicating reduced fertility.

"OPU" as used herein stands for "Ovum Pick Up." OPU is, among other things, a veterinary procedure that can be used in horses. This technique involves collecting unfertilized eggs (oocytes) directly from the follicles of a mare's ovaries. This is usually done using a needle inserted through a transvaginal approach, often under ultrasound guidance. The collected oocytes can then be used for advanced reproductive techniques such as in vitro fertilization (IVF) or intracytoplasmic sperm injection (ICSI). This makes it possible to produce offspring from valuable or genetically important mares that would otherwise be unable to breed successfully due to age, health, or other complications. OPU is considered a valuable tool in horse breeding because it offers the possibility to collect multiple oocytes from a mare in one season, which can increase the efficiency and success of breeding programs. OPU is a method also used, for example, to keep sport mares in competition while still producing embryos from those sport mares.

Fertilization can be done, for example, by means of Intracytoplasmic Sperm Injection (ICSI) or In Vitro Fertilization (IVF). In the case of performing an IVF treatment, a large number of sperm cells are brought to each oocyte. One of these sperm cells will have to penetrate the oocyte itself in order to achieve fertilization. In ICSI, the procedure is similar to IVF, but the obtained oocytes are *in vitro* fertilized by direct injection of a sperm cell through the ooplasm. In the ICSI procedure, the oocytes are stripped of the surrounding supportive tissue after puncture. This allows for the identification of which oocytes are mature. Only mature oocytes are suitable for injection with a sperm cell. After the oocyte is injected, the nuclei of the oocyte and sperm cell must fuse to achieve fertilization.

The "equids" (Equidae) as used herein, are a family of odd-toed ungulate mammals. Today there exists only one genus, Equus. The equids include four groups: horses (including ponies), donkeys, onagers (Asiatic wild asses), and zebras. In particular, the subject of the equid group concerns a mare.

Quoting numeric intervals by the endpoints includes all integers, fractions, and/or real numbers between the endpoints, including those endpoints.

### Detailed description

The reproductive performance of mares is lower than that of other domesticated animals in terms of fertilization rate.

There are various factors that can contribute to fertility problems in mares, for example endometritis, anovulatory follicles, the age of the mares, and/or the presence of metabolic syndrome. Successful folliculogenesis, oocyte maturation, and ovulation also play an important role.

Various pathogenic factors can lead to failed folliculogenesis, oocyte maturation, and/or ovulation and have serious consequences on fertility. Genetic factors play an important role in this. The maturation of oocytes is, for example, a well-organized, complex process regulated by a large number of genes. Pathogenic variants in these genes, as well as aneuploidy, defects in the mitochondrial genome, and other genetic and epigenetic factors, can lead to unexplained infertility, early pregnancy loss, and recurrent failures of IVF/ICSI programs due to poor ovarian response to stimulation, stagnation of oocyte maturation, poor oocyte quality, failed fertilization, or early embryonic developmental arrest.

The maturation of oocytes or "oocyte maturation" is a complex and vital process used to achieve the full competence required by both the oocyte itself and early embryonic development.

In humans and animals, multiple factors, including epigenetic molecules and various signaling pathways, have been identified and proven to be crucial for this maturation. They not only regulate the maturation of oocytes but also coordinate with each other to ensure good quality of the oocytes, both of which are necessary to guarantee optimal fertility.

Epigenetic processes include, in addition to DNA methylation, multiple post-translational modifications of various proteins in developing follicles and oocytes, including acetylation, phosphorylation, methylation, glycosylation, ubiquitination, and SUMOylation. Histone methylation, for example, correlates with chromatin activity. H3K4 (Histone 3 lysine 4) methylation, for example, is associated with chromatin activation and is mainly found in the promoter regions of active genes, while methylation of H3K9 (Histone 3 lysine 9) or H3K27 (Histone 3 lysine 27) is associated with gene inactivation.

These epigenetic processes have a significant impact on oocyte maturation and oocyte quality. Studies have shown, for example, that in developing oocytes (eggs), the level of H3K4me3 is increased during the transition of the chromatin configuration from the non-surrounded nucleolus type (NSN) to the surrounded nucleolus type (SN), where the latter has better developmental competence after fertilization.

Establishing the correct epigenetic modifications during oocyte maturation and early embryonic development is an important aspect of reproduction. External and internal environmental changes can cause changes in epigenetic modifications in oocytes. Research has shown that the establishment of epigenetic modifications in oocytes is influenced by hormones and the maternal diet. If changes in epigenetic modifications occur in the germline, this could affect embryonic development and the health of the offspring, and this effect could even extend to subsequent generations. Therefore, the association of oocyte quality and epigenetic modifications is crucial for fertility.

In a first aspect, the invention relates to a composition for use in treating ovulatory infertility in equids, the composition comprising a mixture of one or more methyl donors. Methyl donors are substances that can donate methyl groups (-CH3) for various biochemical reactions in the body, including DNA methylation and other methylation processes involved in gene expression and cell metabolism.

As described above, histone methylation correlates with chromatin activity. H3K4 (Histone 3 lysine 4) methylation, for example, is associated with chromatin activation and is mainly found in the promoter regions of active genes, while methylation of H3K9 (Histone 3 lysine 9) or H3K27 (Histone 3 lysine 27) is associated with gene inactivation. Studies have shown that in developing oocytes, the level of H3K4me3 is increased during the transition of the chromatin configuration from the non-surrounded nucleolus type (NSN) to the surrounded nucleolus type (SN), where the latter has better developmental competence after fertilization.

Methyl donors are therefore important for DNA methylation and other methylation processes (such as histone methylation) involved in gene expression and cell metabolism, including gene expression during oocyte maturation. Disrupted methylation affects the quality of oocytes and embryos. As described above, methylation is essential for the proper regulation of gene expression. Disrupted methylation can lead to abnormal activation or suppression of genes crucial for the development and maturation of oocytes, as well as for early embryonic development. The maturation of oocytes is a precisely regulated process in which methylation plays a role. A disruption in this process can lead to the production of lower-quality oocytes, reducing fertility and the chance of successful fertilization.

Furthermore, in the early stages of embryonic development there is a need for epigenetic reprogramming, including extensive changes in DNA methylation patterns. Disrupted methylation during this critical period can disrupt normal development and lead to developmental defects or early embryonic death. Furthermore, methylation also plays a role in the processes leading to the implantation of the embryo in the uterus and the formation of the placenta. Abnormal methylation patterns can affect these processes and reduce the embryo's chances of survival. There is also evidence that disrupted methylation patterns can be passed from mother to offspring, affecting the health and fertility of future generations. Disrupted methylation can affect the integrity of the genome and epigenome, leading to genetic mutations or abnormal epigenetic patterns, with potentially adverse consequences for embryo development.

Thus, disrupted methylation affects not only the quality of oocytes but also the quality of embryos. Methyl donors play a crucial role in the epigenetic management and development of the embryo after fertilization. These compounds provide methyl groups (-CH3) that are essential for the process of DNA methylation, an important form of epigenetic modification. DNA methylation is involved in the regulation of gene expression. After fertilization, extensive epigenetic reprogramming occurs in the embryo, where parental epigenetic markers are erased and new ones are established. Methyl donors are crucial for this process.

Adequate methylation is essential for normal embryonic development. A deficiency of methyl donors can lead to abnormal developmental patterns or birth defects. Methylation also plays a role in the development of the placenta. Proper methylation is necessary for the formation and function of the placenta, which in turn is essential for the nutrition and oxygen supply of the growing embryo. Some genes are "imprinted," meaning they are selectively expressed depending on whether they are inherited from the mother or the father. Methylation is crucial for this process, and disruptions can lead to imprinting disorders. Methylation plays a role in the transgenerational transfer of epigenetic information. The epigenetic state of an embryo can be influenced by the nutritional and health status of the parents, particularly the methylation status. Research also suggests that the methylation status during early development can affect the individual's health later in life, including risks for certain diseases.

Methyl donors such as folic acid, vitamin B12, choline, and betaine play a vital role in supporting healthy embryonic and fetal development.

Other examples of methyl donors include dimethylglycine (DMG) and S-methylmethionine (SMM).

In a preferred embodiment, the methyl donor is betaine (trimethylglycine), and the composition includes betaine and/or its precursor choline or a choline derivative.

In one embodiment, betaine is present in the composition in the form of its precursor choline or a choline derivative.

Choline is found in many foods, mainly in animal products. Sources rich in choline include eggs, milk, and red meat. In one embodiment, choline is present in the composition in the form of choline chloride. Choline chloride is a quaternary ammonium salt of hydrochloric acid, with the chemical formula HOCH₂CH₂N(CH₃)₃Cl. The substance appears as colorless to white hygroscopic crystals, which are soluble in water. Choline chloride is a choline derivative. In a further embodiment, choline chloride is mixed with an amount of silica, such as 50% silica, further referred to as "choline chloride 50%". In one embodiment, an effective dose of the composition includes at least 5 g, 6 g, 7 g, 8 g, 9 g, 10 g, 11 g, 12 g, 13 g, 14 g, 15 g, 16 g, 17 g, 18 g, 19 g, 20 g, 21 g, 22 g, 23 g, 24 g, or 25 g of choline or a choline derivative per 100 g of composition.

In one embodiment, an effective dose of the composition includes 5 g, 6 g, 7 g, 8 g, 9 g, 10 g, 11 g, 12 g, 13 g, 14 g, 15 g, 16 g, 17 g, 18 g, 19 g, 20 g, 21 g, 22 g, 23 g, 24 g, or 25 g of choline or a choline derivative per 100 g of composition. Choline derivatives are known from the prior art and include choline chloride, alpha-glycerophosphocholine, cytidine diphosphate choline, and choline bitartrate.

Other examples of choline derivatives are phosphatidylcholine (a phospholipid found in lecithin, for example, from soy or sunflowers), citicoline (CDP-choline), choline citrate, and sphingomyelin.

In one embodiment, an effective dose of the composition includes at least 10 g, 11 g, 12 g, 13 g, 14 g, 15 g, 16 g, 17 g, 18 g, 19 g, 20 g, 21 g, 22 g, 23 g, 24 g, or 25 g of choline chloride 50% per 100 g of composition. In one embodiment, an effective dose of the composition includes 10 g, 11 g, 12 g, 13 g, 14 g, 15 g, 16 g, 17 g, 18 g, 19 g, 20 g, 21 g, 22 g, 23 g, 24 g, or 25 g of choline chloride 50% per 100 g of composition.

Choline can be oxidized in the liver and kidneys to betaine (trimethylglycine, TMG).

In one embodiment, the composition includes betaine (also known as betaine anhydrous or trimethylglycine (TMG)). Betaine aids in the methylation of proteins, including histones. Additionally, as a methyl donor in the liver, betaine facilitates the (re)methylation of homocysteine to methionine, the precursor of SAMe (S-adenosylmethionine, the universal methyl donor), via the enzyme betaine-homocysteine methyltransferase (BHMT). Choline influences (via betaine/SAMe) the methylation of DNA and histones and thus gene expression, including the gene expression necessary for successful oocyte maturation. Due to its activity as a methyl donor, TMG can further ensure that the methyl pool in our body remains optimal, especially when supplemented with niacin (vitamin B3), known to be a methyl consumer. An imbalance can lead to changes in the methyl donor substrate levels and consequently to a modulation of the DNA methylation pattern and histone methylation. In one embodiment, betaine is derived from the root of Beta Vulgaris (red beet).

In a preferred embodiment, an effective dose of the composition includes at least 5 g of choline (or choline derivative) and at least 8 g of betaine per 100 g of composition. Folate (also known as vitamin B11 or vitamin B9) also facilitates the (re)methylation of homocysteine to methionine.

About 90% of dietary folates contain more than one glutamate group and are pteroylpolyglutamates based on 5-methyl-tetrahydrofolate (5-MTHF). Typically, there are five to seven groups, up to a maximum of eleven, connected by peptide bonds. The most common dietary folates are 5,6,7,8-tetrahydrofolates (THF) and 7,8-dihydrofolate (DHF). Folate is active in the body as (various forms of) tetrahydrofolate. Folate from the diet is therefore reduced to dihydrofolate and then further reduced to tetrahydrofolate. The enzyme dihydrofolate reductase catalyzes both conversions.

Folate provides the methyl group needed to make methylcobalamin, a substance necessary to break down and convert harmful homocysteine into the amino acid methionine. As discussed above, methionine is the precursor of the universal methyl donor SAMe (S-adenosylmethionine), involved in the methylation of DNA and histones, among other things.

Vitamin B6, vitamin B12, and betaine are also needed in homocysteine metabolism and are therefore important for DNA methylation patterns and histone methylation.

In a preferred embodiment, the composition further comprises vitamin B9 (folate or vitamin B11), vitamin B12, vitamin B6, and/or vitamin E. In one embodiment, the composition comprises between 0.05 and 2 weight percent vitamin B9. In one embodiment, the composition comprises between 0.01 and 1 weight percent vitamin B12. In one embodiment, the composition comprises between 0.5 and 5 weight percent vitamin E.

The gonadal hormone 17β-estradiol (E2) and its receptors are important regulators of gene transcription by binding to estrogen-responsive elements in the genome. In addition to the classical genomic action, E2 regulates gene transcription via modification of epigenetic markings on DNA and histone proteins. Depending on the reaction partner, the ligand bound estrogen receptor (ER) promotes DNA methylation in the promoter or enhancer regions. Additionally, ERs are important regulators of passive and active DNA demethylation and, in collaboration with various histone-modifying enzymes and chromatin remodeling complexes, alter gene transcription.

However, it can happen that the hormone balance in a subject is disturbed, leading to infertility. Among other things, estrogen metabolism can be disrupted. The estrogen balance plays an important role in fertility.

Estrogens belong to the group of steroid hormones. Steroid hormones are synthesized from cholesterol. Through enzymatic conversions, cholesterol is transformed into progesterone and further into various androgens such as testosterone. Under the influence of FSH, granulosa cells convert testosterone into the biologically active estrogen 17β-estradiol (E2). Estrogens mainly ensure that the possibility of fertilizing the oocyte is optimized. The estrogen concentration begins to rise about 6 to 8 days before ovulation. Around two days before ovulation, a clear estrogen peak can be observed. The rising estrogen concentration is caused by follicular growth and is externally recognizable by the typical estrus behavior of the mare, also known as heat. Just before ovulation, there is a rapid decline in estrogen concentration. At the time of ovulation, the oocyte (egg cell) is released from the follicle, and a corpus luteum (CL) is formed.

Follicles can sometimes end differently than resulting in a CL after ovulation. Anovulatory follicles are follicles that become abnormally large (5-15 cm) and often remain present for 3-4 days. Ovulation of these follicles does not occur. Such a hemorrhagic anovulatory follicle (HAF) does not rupture and empty and has no contact with the ovulation fossa. Anovulatory follicles result from a very slow process where there is no clear transition from follicle to ovulation, but luteinization of the follicle still occurs. The CL that results is responsive to prostaglandins. If a mare experiences this once, it is very likely to occur again during the breeding season.

The incidence of HAFs is about 5% and 20% of estrous cycles during the early and late ovulatory seasons, respectively. The structures are more common in older mares (e.g., >20 years), tend to recur in individuals, and occur most frequently during the late follicular phase.

During a study on the echotexture of the follicle wall in ponies, HAFs formed during 3 of 56 (5%) estrous cycles early in the ovulatory season (March to May) and in 5 of 23 (23%) late in the season (September and October). No differences were found between the two groups (normal ovulation versus HAF) in systemic concentrations of progesterone, luteinizing hormone (LH), and follicle-stimulating hormone (FSH) on day -4 to 2 ("day 0" is the day of ovulation, whether or not HAF formation occurs), but estradiol was elevated in the HAF group on day -3. Estradiol concentrations were elevated several days before the failure to ovulate and the follicle wall was more vascularized on day -1. Therefore, an increased estradiol concentration may play a role in the formation of HAFs.

The estrogen receptor (ER) is a member of the steroid/thyroid superfamily of proteins that function as hormone-inducible transcription factors. Two ER gene products, namely ER alpha (ERo) and ER beta (ERβ), mediate the action of estrogens in target tissues and thus regulate the estrogenic effects on blood vessels, reproductive tissues, bones, liver, and brain. Ligands, such as estradiol, bind to the ligand-binding domain (LBD) of ER and induce conformational changes that lead to high-affinity binding of ER homodimers to estrogen response elements (ERE) on genes. ER alpha and beta are homologous in some DNA-binding domains, but other areas of the proteins share little homology. The two receptors exhibit significant differences in their pattern of cell-specific expression, ligand-binding affinities, and target genes.

In a preferred embodiment, the composition further comprises glucobrassicin or a source of glucobrassicin. Indole-3-carbinol (I3C) is a breakdown product of glucosinolates, such as glucobrassicin. Various glucosinolates are present in cruciferous vegetables, and when these vegetables are consumed and crushed (such as by chewing), an enzyme called myrosinase is activated. Myrosinase breaks down glucosinolates into various products, including indole-3-carbinol. When I3C enters the acidic environment of the stomach, it is further broken down and condensed to form diindolylmethane (DIM).

Like isothiocyanates, these belong to the group of indirect antioxidants, stimulate the production of detoxifying liver enzymes, and are thus involved in the elimination of harmful free radicals that can damage tissues and DNA. However, they also influence estrogen metabolism, partly because they function as a negative regulator of estrogen by inhibiting ERo signaling and altering cytochrome P450-mediated estrogen metabolism.

Glucobrassicin and metabolites such as DIM can balance estrogen metabolism by increasing the ratio of 2-hydroxyestrone to 16-hydroxyestrone and by stimulating the ER beta receptor and inhibiting the ER alpha receptor. 16-hydroxyestrone is associated with estrogen stimulation, whereas 2-hydroxyestrone competes with estradiol for binding to the estrogen receptor. This results in fewer free estrogens in the bloodstream, balancing the estrogen level. Balancing the estrogen level with the composition of the current invention can treat various conditions related to a disturbed estrogen level (such as estrogen dominance, hemorrhagic anovulatory follicles, etc.).

Indole-3-carbinol, DIM, and ICZ namely bind and activate the Aryl hydrocarbon receptor (AhR). AhR increases the gene expression of the phase I enzyme CYP1A1 (Cytochrome P450, family 1, subfamily A, polypeptide 1) and the phase II enzymes glutathione S-transferase and oxidoreductases.

CYP1A1 is a protein encoded by the CYP1A1 gene in humans. The protein is a member of the cytochrome P450 superfamily of enzymes. CYP1A1 is responsible for the 2-hydroxylation of estrone, one of the two main competing hydroxylation pathways of estrogen metabolism, leading to increased levels of 2-hydroxyestrone. As described above, 16-hydroxyestrone is associated with estrogen stimulation, while 2-hydroxyestrone competes with estradiol for binding to the estrogen receptor. Consequently, I3C can act as a phytoestrogen by inducing a competing metabolic pathway that increases the production of 2-hydroxyestrone and thus reduces the substrate available for the production of 16-hydroxyestrone.

Indole-3-carbinol is a negative regulator of ERo signaling. In addition to estrogen metabolism by CYP1A1, indole-3-carbinol and its metabolites also affect ER signaling through two different mechanisms. First, indole-3-carbinol and DIM can bind to and inhibit the activity of ER, reducing the cellular and biochemical effects of estradiol in estrogen-sensitive cells and tissues.

Second, indole-3-carbinol and DIM could suppress the expression of ERo in certain cells, which could be attributed to the ability of these indole derivatives to bind to the nuclear AhR. Moreover, it was reported that indole-3-carbinol increases the binding of ERb to the estrogen response element, resulting in a significantly higher ERβ/ERα ratio.

In a preferred embodiment, the composition also includes sulforaphane and/or one or more plant components as a source of sulforaphane.

Sulforaphane, a type of isothiocyanate (sulfur-containing compounds mainly in cruciferous vegetables) in broccoli, has been of considerable scientific interest in recent years. In the plant, sulforaphane is only found bound, as part of sulforaphane glucosinolate (glucoraphanin). The seeds and sprouts of broccoli are richest in these substances. On average, sprouts contain twenty to fifty times higher concentrations of glucoraphanin than in the mature broccoli plant, with even higher concentrations in broccoli seeds. Glucoraphanin can be converted into sulforaphane by two pathways. Via the enzyme myrosinase released during crushing and chewing or via conversion in the gut flora. At a pH higher than 5, more than 80% of glucoraphanin is namely converted into sulforaphane.

In one embodiment, the composition contains broccoli sprouts as a source of glucobrassicin and/or sulforaphane.

Sulforaphane is one of the most potent known detoxifying substances and protects cells against various diseases, particularly those where DNA can be irreversibly damaged, through the aforementioned mechanism.

In a further preferred embodiment, the broccoli sprouts are present in the form of a broccoli extract. In a further preferred embodiment, the broccoli extract contains at least 10 weight percent glucosinolates, based on the total weight of the broccoli extract. In embodiments, the broccoli extract contains at least 11, 12, 13, 14, 15, 16, 17, 18, 19, 20, 21, 22, 23, 24 or weight percent glucosinolates, based on the total weight of the broccoli extract. In embodiments, the broccoli extract contains 10, 11, 12, 13, 14, 15, 16, 17, 18, 19, 20, 21, 22, 23, 24 or weight percent glucosinolates, based on the total weight of the broccoli extract.

In a preferred embodiment, an effective dose of the composition includes at least 15 g of broccoli extract per 100 g of composition. In embodiments, an effective dose of the composition includes at least 16, 17, 18, 19, 20, 21, 22, 23, 24, or 25 g of broccoli extract per 100 g of composition. In embodiments, an effective dose of the composition includes 15.0; 15.5; 16.0; 16.5; 17.0; 17.5; 18.0; 18.5; 19.0; 19.5; 20.0; 20.5; 21.0; 21.5; 22.0; 22.5; 23.0; 23.5; 24.0; 24.5 or 25.0 g of broccoli extract per 100 g of composition.

In one embodiment, the extract also includes the activated enzyme myrosinase.

In one embodiment, the composition further comprises lignans or a source of lignans.

The richest sources of lignans are linseed (especially the husk) and soy. But grains, legumes, garlic, pistachios, pumpkin seeds, seaweed, and chestnuts are also good suppliers. In a preferred embodiment, the composition contains linseed extract as a source of lignans. In a further preferred embodiment, the linseed extract contains at least 20 weight percent lignans, based on the total weight of the linseed extract. In one embodiment, the linseed extract contains at least 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 weight percent lignans, based on the total weight of the linseed extract. In one embodiment, the linseed extract contains 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 weight percent lignans, based on the total weight of the linseed extract.

Lignans are especially known for their hormone-binding capacity. They increase the production of so-called "SHBG" ("sex hormone-binding globulins"). This substance is produced by the liver and ensures that there are not too many free hormones present in the body. By binding estrogens, the free concentration of the latter can decrease, allowing the estrogen balance to be restored.

Dietary lignans include lignanolactones and lignanodiols and are mainly found as glycosides in nature. These lignans are also capable of inhibiting aromatase (CYP450arom) and 17β-hydroxysteroid dehydrogenase (17β-HSD) and thereby reducing the production of 17β-estradiol (E2).

Aromatase (CYP450arom), estrogen synthetase, or estrogen synthase, is an enzyme responsible for an important step in the biosynthesis of estrogens. It is a member of the cytochrome P450 superfamily, a group of mono-oxygenases that catalyze steps in the synthesis of many steroids. Aromatase is specifically responsible for converting androgens into estrogens by forming a benzene ring. It converts the aliphatic androgens testosterone and androstenedione into the aromatic estrogens estradiol (E2) and estrone (E1), respectively. Inhibition of aromatase thus results in reduced conversion and lower concentrations of E2 and E1. 17β-HSD (type I and II) regulate the balance between E2 and the biologically less active E1. Inhibition of 17β-HSD reduces the production of the more active 17β-estradiol (E2). By inhibiting aromatase and 17β-hydroxysteroid dehydrogenase, the estrogen balance can be restored.

In a preferred embodiment, an effective dose of the composition includes at least 5 g of linseed extract per 100 g of composition. In embodiments, an effective dose of the composition includes at least 6, 7, 8, 9, 10, 11, 12, 13, 14, 15, 16, 17, 18, 19, 20, 21, 22, or 23 g of linseed extract per 100 g of composition. In embodiments, an effective dose of the composition includes 5.0; 5.5; 6.0; 6.5; 7.0; 7.5; 8.0; 8.5; 9.0; 9.5; 10.0; 10.5; 11.0; 11.5; 12.0; 12.5; 13.0; 13.5; 14.0; 14.5; 15.0; 15.5; 16.0; 16.5; 17.0; 17.5; 18.0; 18.5; 19.0; 19.5; 20.0; 20.5; 21.0; 21.5; 22.0; 22.5 or 23.0 g of linseed extract per 100 g of composition.

In one embodiment, the composition further comprises cysteine.

"Cysteine" preferably refers to L-cysteine and also includes salts of cysteine, such as L-Cysteine hydrochloride (HCL) anhydrous.

L-cysteine is a precursor of glutathione. The intracellular cysteine content is the determining factor for the final amount of intracellular glutathione. Glutathione is present in almost all cells at high concentrations and is one of the most important antioxidants in the body. Therefore, it also plays an important role in protecting DNA and genes involved in oocyte maturation.

In a preferred embodiment, an effective dose of the composition includes at least 10 g of cysteine per 100 g of composition. In one embodiment, an effective dose of the composition includes 10 g of cysteine per 100 g of composition. In one embodiment, an effective dose of the composition includes 11 g of cysteine per 100 g of composition. In one embodiment, an effective dose of the composition includes 12 g of cysteine per 100 g of composition. In one embodiment, an effective dose of the composition includes 13 g of cysteine per 100 g of composition. In one embodiment, an effective dose of the composition includes 14 g of cysteine per 100 g of composition. In one embodiment, an effective dose of the composition includes 15 g of cysteine per 100 g of composition. In one embodiment, an effective dose of the composition includes 16 g of cysteine per 100 g of composition. In one embodiment, an effective dose of the composition includes 17 g of cysteine per 100 g of composition. In one embodiment, an effective dose of the composition includes 18 g of cysteine per 100 g of composition. In one embodiment, an effective dose of the composition includes 19 g of cysteine per 100 g of composition. In one embodiment, an effective dose of the composition includes 20 g of cysteine per 100 g of composition. In one embodiment, an effective dose of the composition includes 21 g of cysteine per 100 g of composition. In one embodiment, an effective dose of the composition includes 22 g of cysteine per 100 g of composition. In one embodiment, an effective dose of the composition includes 23 g of cysteine per 100 g of composition. In one embodiment, an effective dose of the composition includes 24 g of cysteine per 100 g of composition. In one embodiment, an effective dose of the composition includes 25 g of cysteine per 100 g of composition.

In a preferred embodiment, an effective dose of the composition includes:
- at least 10 g of cysteine per 100 g of composition;
- at least 5 g of choline or choline derivative and at least 8 g of betaine per 100 g of composition;
- at least 15 g of broccoli extract per 100 g of composition;
- at least 5 g of linseed extract per 100 g of composition.

In a preferred embodiment, the composition includes:
- Between 15 and 25 weight percent broccoli extract;
- Between 5 and 15 weight percent linseed extract;
- Between 10 and 15 weight percent cysteine;
- Between 5 and 15 weight percent choline or choline derivative;
- Between 8 and 12 weight percent betaine;
- Between 0.05 and 2 weight percent vitamin B9;
- Between 0.01 and 1 weight percent vitamin B12;
- Between 0.5 and 5 weight percent vitamin E.

In a preferred embodiment, the composition includes:
- 22.5 weight percent broccoli extract;
- 17.5 weight percent linseed extract;
- 12 weight percent cysteine;
- 12 weight percent choline chloride 50%;
- 10 weight percent betaine;
- 0.2 weight percent vitamin B9;
- 0.08 weight percent vitamin B12;
- 2.82 weight percent vitamin E.

The composition is optimized such that the ingredients have a synergistic effect on optimizing the fertility of a subject, preferably an animal such as a horse. More specifically, it has been found that the composition has an optimal effect on the estrogen balance and methylation processes involved in gene expression and cell metabolism, which in turn positively influences the gene transcription of genes involved in the aforementioned oocyte maturation.

Compositions according to the present description may additionally and/or optionally further comprise one or more free amino acids or peptides, such as lysine, methionine, histidine, leucine, isoleucine, alanine, phenylalanine, asparagine, arginine, beta-alanine, aspartic acid, tryptophan, proline, threonine, selenocysteine, serine, taurine, tyrosine, valine, glycine, glutamine, glutamic acid, ornithine, carnosine, L-carnitine, glutathione. Compositions according to the present description may additionally and/or optionally further comprise one or more minerals selected from the group of calcium, sodium, potassium, phosphorus, chromium, vanadium, magnesium, zinc, manganese, selenium, copper, molybdenum, boron, vanadium, iron (and/or derivatives) or a combination thereof.

According to one embodiment of the invention, the composition comprises an orally administrable dosage form. Examples of orally administrable dosage forms include, but are not limited to, a tablet, capsule, powder that can be dispersed in a beverage, a liquid such as a solution, suspension or emulsion, a soft gel/chewable capsule, a paste, elixir, syrup, drops, bar, beverage, chewable tablet, a chewable bar, or another suitable dosage form known in the art. Preferably, the composition is formulated in powder form, as a tablet, a capsule, or a granule. Moreover, the orally administrable dosage forms can be formulated for immediate release, sustained release, or delayed release.

In a preferred form, the composition is a powder, and a daily dose is preferably between 2 and 30 grams, more preferably between 5 and 25 grams, more preferably between 10 and 20 grams, such as 15 grams. The composition can be packaged in bulk or per daily dose, in a sachet or stick.

Compositions according to the present description may additionally and/or optionally further comprise one or more excipients. These excipients are selected from the following group: microcrystalline cellulose, crystalline cellulose, lactose, corn starch, sucrose, glucose; binders such as tragacanth, gum arabic, corn starch, gelatin, polyvinyl alcohol, polyvinyl ether, ethylcellulose, methylcellulose, shellac, hydroxypropylcellulose, hydroxypropyl starch, polyvinylpyrrolidone; swelling agents such as corn starch, pregelatinized starch, alginic acid, dextrin; lubricants such as magnesium stearate; flowability enhancers such as fine silicon dioxide; lubricants such as glyceryl fatty acid ester, magnesium stearate, talc, polyethylene glycol, silica, hydrogenated vegetable oil; sweeteners such as sucrose, lactose, aspartame, acesulfame-K, sucralose, monatin, stevia, saccharin, and the like; flavors to be used for various foods such as peppermint, vanilla flavor, cherry, raspberry ketone, and the like; preservatives such as paraoxybenzoates, chlorobutanol, benzyl alcohol, sorbic acid; an antioxidant, such as sulfite, ascorbic acid, vitamin E, butylated hydroxytoluene, sodium sulfite; and/or coating agents such as shellac, sucrose, gelatin, and hydroxypropylcellulose.

Compositions and formulations according to the present invention may additionally and/or optionally further comprise one or more carrier materials, such as corn starch, acacia, gelatin, malt, tragacanth, microcrystalline cellulose, kaolin, dicalcium phosphate, calcium carbonate, sodium chloride, or alginic acid; disintegrants including microcrystalline cellulose or alginic acid; binders including acacia, methylcellulose, ethylcellulose, sodium carboxymethylcellulose, polyvinylpyrrolidone, or hydroxypropylmethylcellulose; and lubricating agents such as magnesium stearates, stearic acid, silicone fluid, talc, oils, waxes, or colloidal silica.

Compositions and formulations according to the present invention may additionally and/or optionally further comprise one or more aqueous or non-aqueous carriers, diluents, fillers, binders, wetting agents, disintegrants, dissolution retardants, absorption accelerators, wetting agents, absorbents, or lubricants, solvents, or carriers, including water-ethanol, polyols (propylene glycol, polyethylene glycol, or glycerol), suitable mixtures thereof, vegetable oils (such as olive oil), and organic esters such as ethyl oleate.

Furthermore, the compositions and formulations according to the present description may include stabilizers, solubilizing agents, suspending agents, emulsifiers, soothing agents, buffers, preservatives, isotonic agents, or antibacterial and antifungal agents.

As described above, it has been found that the composition has an optimal effect on the methylation processes involved in gene expression and cell metabolism, which in turn positively influences the gene transcription of genes involved in the aforementioned oocyte maturation. The current invention is capable of treating ovulatory sub- or infertility by optimizing the process of oocyte maturation to increase the number of oocytes and/or the quality of the oocytes. The current invention makes it possible, for example, to increase the number and/or quality of the unfertilized oocytes that are collected directly from the follicles of a mare's ovaries through OPU. In addition to treating ovulatory disorders that play a role in *in vivo* fertilization, the current invention also makes it possible to optimize the OPU procedure and subsequent ex *vivo* fertilization due to the increased number of oocytes and the improved quality of the oocytes that can be collected. This makes it possible to produce offspring from valuable or genetically important mares that would otherwise be unable to breed successfully due to age, sports career, health, or other complications.

In a second aspect, the invention therefore relates to a method for collecting one or more unfertilized oocytes from the follicles of the ovaries of a subject of the equid group, wherein prior to collecting the one or more unfertilized oocytes, a composition is administered to the subject, the composition comprising a mixture of one or more methyl donors.

The composition is as described above and can occur in any of the embodiments described above. As described above, the composition is optimized such that the ingredients have a synergistic effect on optimizing the fertility of a subject, preferably an animal such as a horse. More specifically, it has been found that the composition has an optimal effect on the estrogen balance and methylation processes involved in gene expression and cell metabolism, including the gene transcription of genes involved in oocyte maturation. As described above, the composition according to the current invention contributes to improved oocyte maturation, increasing the number of follicles that can be punctured and/or the number of immature oocytes that can be harvested and/or the number of matured oocytes (the success rate of the maturation of the collected oocytes is increased, visible by the presence or absence of a polar body after 24 hours in *in vitro* culture).

The aim of collecting unfertilized oocytes is generally to fertilize them afterwards.

In a further aspect, the invention therefore also relates to a method for ex *vivo* fertilizing one or more oocytes of a subject, the method comprising:
- collecting one or more unfertilized oocytes from the follicles of the ovaries of the subject;
- performing ICSI (Intracytoplasmic Sperm Injection) and/or IVF (*in vitro* fertilization) for fertilizing the one or more collected oocytes,
wherein prior to collecting the one or more unfertilized oocytes, a composition is administered to the subject, the composition comprising a mixture of one or more methyl donors.

The current invention thus makes it possible to optimize the OPU procedure and subsequent ex *vivo* fertilization and early embryonic development. There are various factors that can contribute to less favorable results in Ovum Pick Up (OPU) procedures in mares. These can relate to the mare itself as well as the conditions and techniques used during the procedure.

The age and fertility status of the mare play a role: older mares or mares with a history of fertility problems may produce fewer and/or lower-quality oocytes. Conditions such as cysts or other abnormalities in the ovaries can also affect the quality and number of available oocytes. The response to hormonal stimulation can also vary greatly between mares. Some mares may not respond optimally to treatments intended to stimulate follicle growth. The natural reproductive cycle of mares is also seasonal, which can result in variations in the availability and quality of oocytes throughout the year.

Stress, both physical and psychological, can affect the mare's reproductive function. Good overall health and well-being are essential for optimal reproductive performance. Malnutrition or nutrient imbalance can also negatively impact the mare's fertility and oocyte quality. Certain medications or previous medical treatments can impact the mare's fertility. Mares that have previously received medications to suppress the ovulatory cycle (Regumate) may experience fertility problems afterward.

The composition is as described above and can occur in any of the embodiments described above. In one embodiment, the composition further comprises glucobrassicin or a source of glucobrassicin, sulforaphane and/or one or more plant components as a source of sulforaphane, lignans or a source of lignans, and/or cysteine. As described above, the methyl donor can be betaine, and betaine can be present in the composition in the form of its precursor choline or a choline derivative. The composition can contain broccoli sprouts as a source of glucobrassicin and/or sulforaphane, where the broccoli sprouts are present in the form of a broccoli extract containing at least 10 weight percent glucosinolates, based on the total weight of the broccoli extract. The composition can contain linseed extract as a source of lignans, where the linseed extract contains at least 20 weight percent lignans, based on the total weight of the linseed extract.

The components of the composition can also occur in the concentrations described above.

As described above, the composition according to the current invention makes it possible to optimize the OPU procedure and subsequent ex *vivo* fertilization and early embryonic development. Various parameters during the OPU procedure can be optimized using the current invention. Among other things, the number and size of the follicles and the quality of the follicles on the mare's ovaries can be optimized. The actual number of oocytes retrieved during the OPU is a very important parameter, which can also be compared to the expected number based on follicle assessment. The morphological quality of the obtained oocytes can also be optimized and is assessed under a microscope. This includes aspects such as the clarity of the cytoplasm, the visibility of the nucleus, and the overall integrity of the oocyte.

As described above, the composition according to the current invention contributes to improved oocyte maturation, increasing the number of follicles that can be punctured and/or the number of immature oocytes that can be harvested and/or the number of matured oocytes (the success rate of the maturation of the collected oocytes is increased, visible by the presence or absence of a polar body after 24 hours in *in vitro* culture) and/or the number of fertilized oocytes that undergo successful division (formation of a blastomere) and/or further develop to the blastocyst stage after fertilization.

The composition can be administered via any route, such as intravenously, orally, rectally, vaginally, transmucosally, sublingually, subdurally, nasally, intramuscularly, subcutaneously, intradermally, intramedullary injection, intrathecally, intraventricularly, intraperitoneally, intranasally, intracerebroventricularly (ICV), ophthalmically, and intraocularly. In a preferred embodiment, the composition is administered orally.

The composition can be administered during any period prior to *in vivo* fertilization (where coverage by a stallion or insemination of the mare takes place) or prior to collecting the unfertilized oocytes, and this according to any regime. The composition can be administered lifelong, for 5 years, 4 years, 3 years, 2 years, 1 year, 24 weeks, 23 weeks, 22 weeks, 21 weeks, 20 weeks, 19 weeks, 18 weeks, 17 weeks, 16 weeks, 15 weeks, 14 weeks, 13 weeks, 12 weeks, 11 weeks, 10 weeks, 9 weeks, 8 weeks, 7 weeks, 6 weeks, 5 weeks, 4 weeks, 3 weeks, 2 weeks, 1 week, or less than 1 week prior to *in vivo* fertilization or the collection of unfertilized oocytes. The administration of the composition can be started between 1 year and 24 weeks, between 24 and 23 weeks, between 23 and 22 weeks, between 22 and 21 weeks, between 21 and 20 weeks, between 20 and 19 weeks, between 19 and 18 weeks, between 18 and 17 weeks, between 17 and 16 weeks, between 16 and 15 weeks, between 15 and 14 weeks, between 14 and 13 weeks, between 13 and 12 weeks, between 12 and 11 weeks, between 11 and 10 weeks, between 10 and 9 weeks, between 9 and 8 weeks, between 8 and 7 weeks, between 7 and 6 weeks, between 6 and 5 weeks, between 5 and 4 weeks, between 4 and 3 weeks, between 3 and 2 weeks, between 2 and 1 week, or less than 1 week prior to *in vivo* fertilization or the collection of unfertilized oocytes. The composition can be administered monthly, weekly, daily, or multiple times per day. In one embodiment, the composition is administered weekly to the mare for a period of 2-4 months prior to *in vivo* fertilization or the collection of unfertilized oocytes. In a preferred embodiment, the composition is administered daily to the mare for a period of 2-4 months prior to *in vivo* fertilization or the collection of unfertilized oocytes. In one embodiment, the composition is administered multiple times (e.g., 2 or 3 times) per day to the mare for a period of 2-4 months prior to *in vivo* fertilization or the collection of unfertilized oocytes. In a preferred embodiment, the composition is administered daily for a period of at least 3 months prior to *in vivo* fertilization or the collection of unfertilized oocytes. In a further preferred embodiment, the composition is administered orally by means of a powder added to the mare's feed.

In a further aspect, the invention relates to a feed additive for mares comprising:
- Between 15 and 25 weight percent broccoli extract;
- Between 5 and 23 weight percent linseed extract;
- Between 10 and 15 weight percent cysteine;
- Between 5 and 15 weight percent choline or choline derivative;
- Between 8 and 12 weight percent betaine.

In a preferred embodiment, an effective dose of the feed additive includes:
- at least 10 g of cysteine per 100 g of composition;
- at least 5 g of choline or choline derivative and at least 8 g of betaine per 100 g of composition;
- at least 15 g of broccoli extract per 100 g of composition;
- at least 5 g of linseed extract per 100 g of composition.

In a preferred embodiment, the feed additive includes:
- Between 15 and 25 weight percent broccoli extract;
- Between 5 and 23 weight percent linseed extract;
- Between 10 and 15 weight percent cysteine;
- Between 5 and 15 weight percent choline or choline derivative;
- Between 8 and 12 weight percent betaine;
- Between 0.05 and 2 weight percent vitamin B9;
- Between 0.01 and 1 weight percent vitamin B12;
- Between 0.5 and 5 weight percent vitamin E.

In a preferred embodiment, the composition includes:
- 22.5 weight percent broccoli extract;
- 17.5 weight percent linseed extract;
- 12 weight percent cysteine;
- 12 weight percent choline chloride 50%;
- 10 weight percent betaine;
- 0.2 weight percent vitamin B9;
- 0.08 weight percent vitamin B12;
- 2.82 weight percent vitamin E.

The components in the feed additive are the same as the composition described above and can occur in any of the embodiments described above. The feed additive is optimized such that the ingredients have a synergistic effect on optimizing the fertility of a subject, preferably an animal such as a horse. More specifically, it has been found that the feed additive has an optimal effect on the estrogen balance and on methylation processes involved in gene expression and cell metabolism, including the gene transcription of genes involved in oocyte maturation, an important process with a significant impact on the fertility of the animal.

In what follows, the invention will be described by way of non-limiting examples illustrating the invention, and which are not intended to and should not be interpreted as limiting the scope of the invention.

### EXAMPLES

### EXAMPLE 1:

A study was set up to test the effects of the feed additive according to an embodiment of the current invention in 8 mares undergoing OPU (Ovum Pick Up). The composition/feed additive includes:
- 22.5 weight percent broccoli extract made from broccoli sprouts, where the broccoli extract contains at least 10 weight percent glucosinolates (based on the total weight of the broccoli extract)
- 17.5 weight percent linseed extract, where the linseed extract contains at least 20 weight percent lignans (based on the total weight of the linseed extract)
- 12 weight percent L-Cysteine hydrochloride (HCL) anhydrous
- 12 weight percent choline chloride mixed with 50% silica ("choline chloride 50%")
- 10 weight percent betaine anhydrous
- 0.2 weight percent vitamin B9
- 0.08 weight percent vitamin B12
- 2.82 weight percent vitamin E
based on the total weight of the composition/feed additive.

"Ovum Pick Up" is a technique where oocytes are directly collected from the mare's ovaries to be fertilized later. In this experiment, fertilization is done by Intracytoplasmic Sperm Injection (ICSI).

For three months prior to the OPU procedure, 15 g of the feed additive is added daily to the mares' feed. Three months after the start of daily administration, an OPU procedure is performed, and the collected oocytes are *in vitro* fertilized by performing ICSI. The effects of the feed additive are evaluated by quantifying the number of follicles that can be punctured (#follicles), the number of oocytes obtained from this (#oocytes), the number of oocytes with successful maturation that can be injected with sperm (#oocytes injected), the number of fertilized oocytes that have undergone successful division (#cleaved), and the number of fertilized oocytes that successfully develop into a blastocyst (#embryos). These values are compared with the values obtained for these parameters in previous OPU-ICSI procedures in the same mare where the feed additive according to an embodiment of the current invention was not administered. Successful maturation of the oocyte is confirmed by the presence of a polar body after 24 hours in culture.

The results of this study are shown in Table 1 below. The results show that the feed additive according to an embodiment of the current invention has a favorable effect on the number of follicles that can be punctured. Six of the eight mares have a higher average number of puncturable follicles when the feed additive according to an embodiment of the current invention was administered compared to when it was not administered. The feed additive according to the current invention supports hormonal balance, partly due to the inclusion of lignans (via the linseed extract) and diindolylmethane (via the broccoli extract). Furthermore, the feed additive also appears to have a favorable effect on the first cell divisions after fertilization (see Table 2). After fertilization, the single-cell zygote divides into two identical cells called blastomeres. As described above, a correct methylation status of the histones and DNA is crucial during this step, and the feed additive according to the current invention provides better developmental competence even after fertilization, partly through the inclusion of methyl donors. In the early stages of embryonic development, there is namely a need for epigenetic reprogramming, including extensive changes in DNA methylation patterns. Disrupted methylation during this critical period can disrupt normal development and lead to developmental defects or early embryonic death.

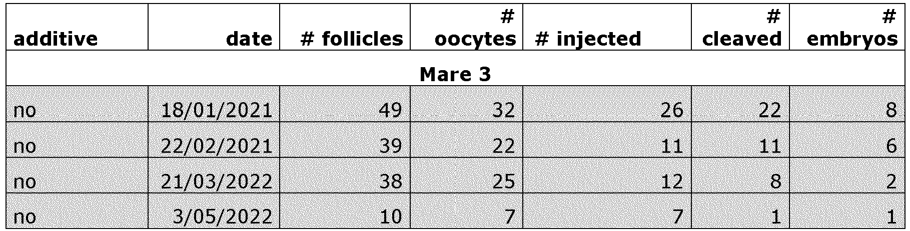

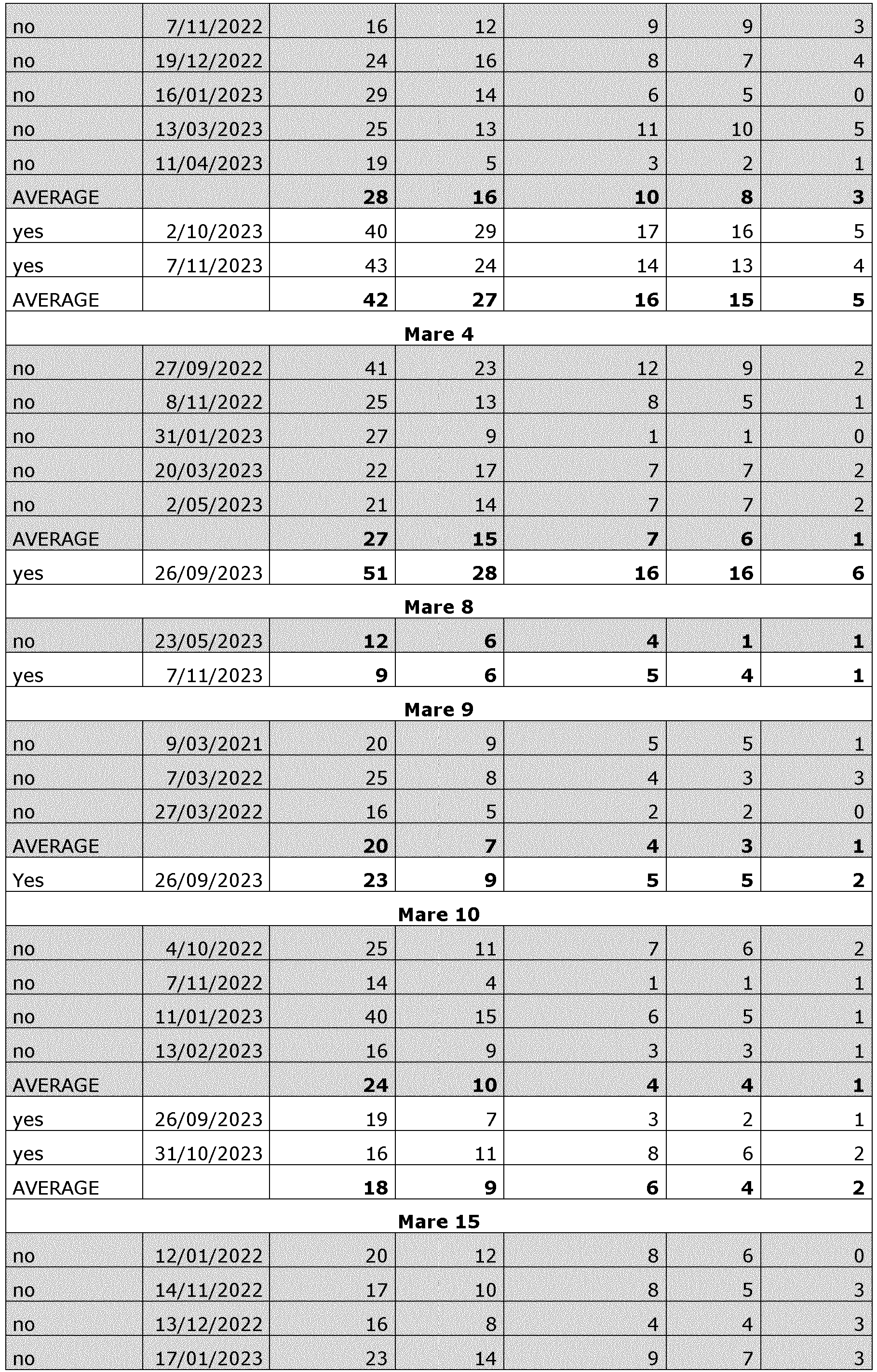

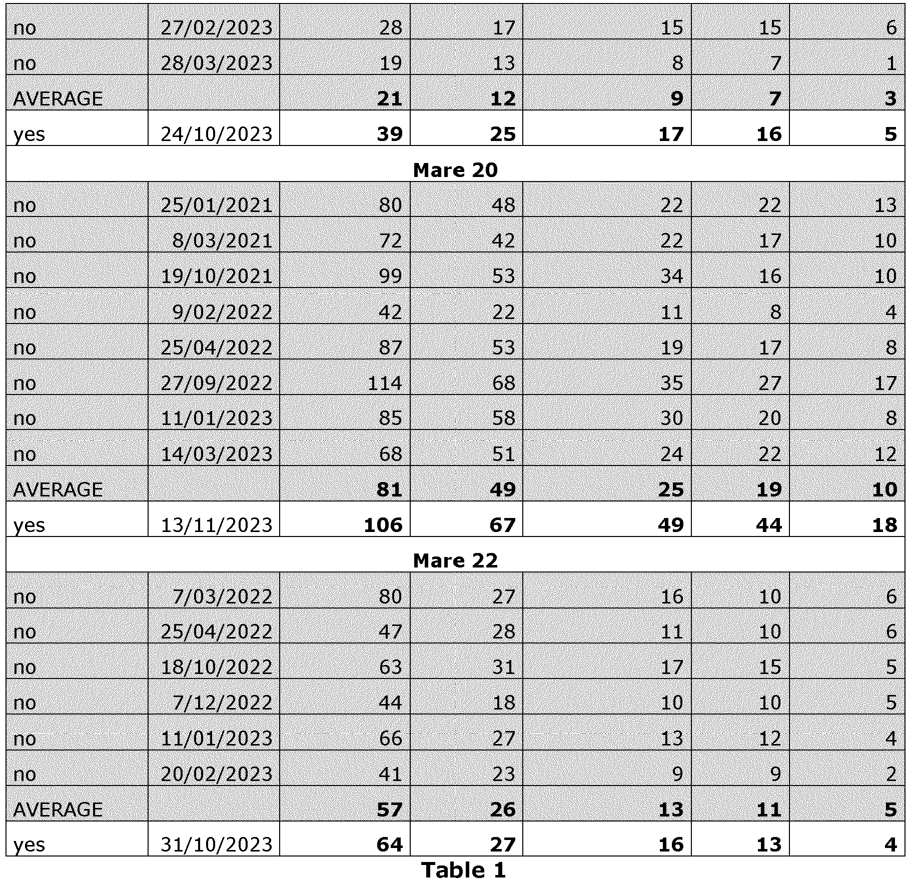

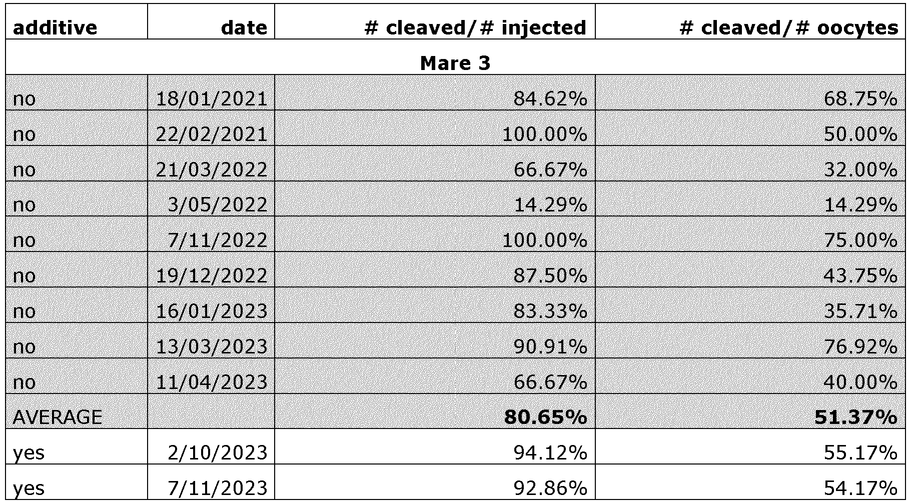

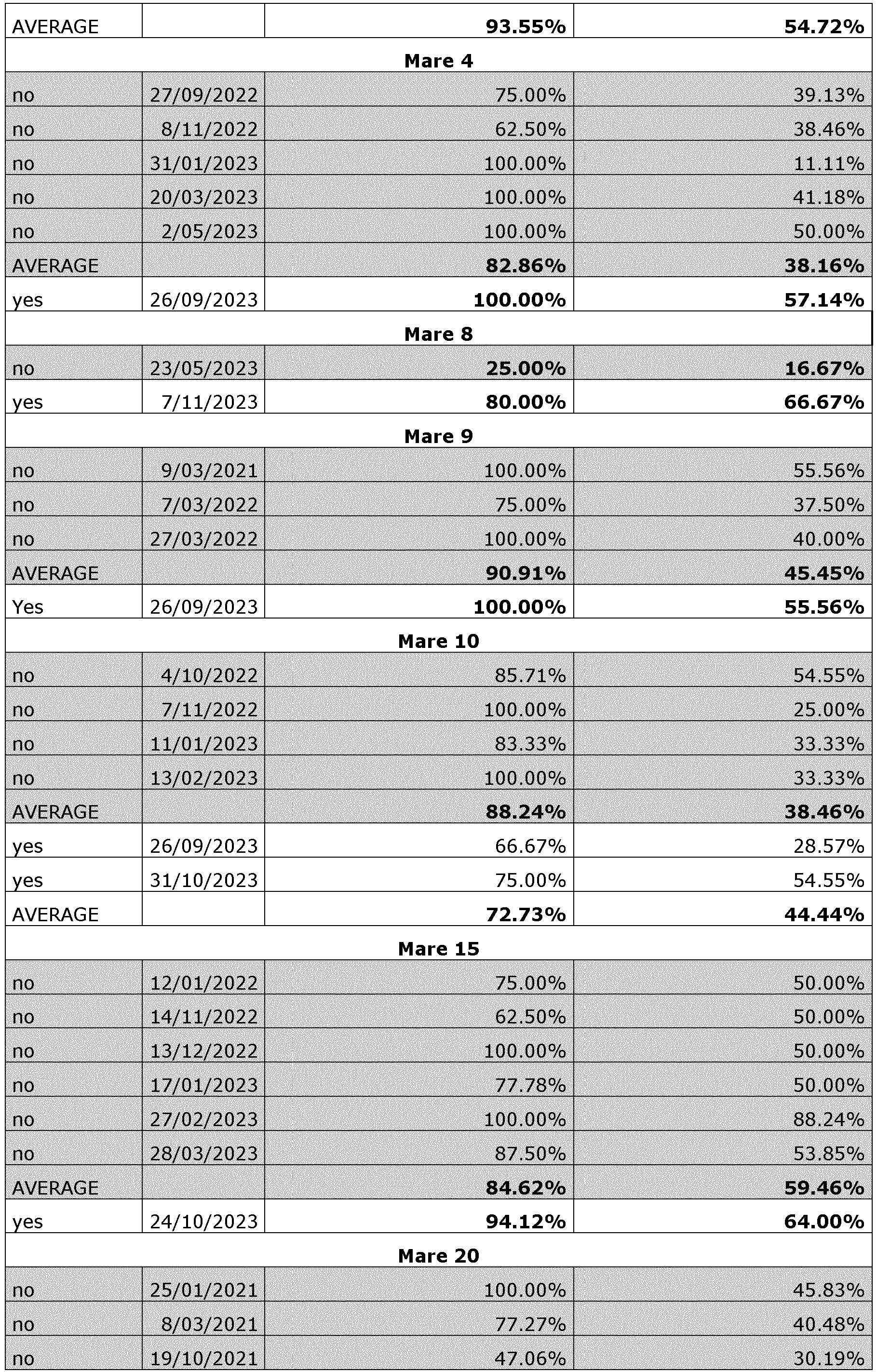

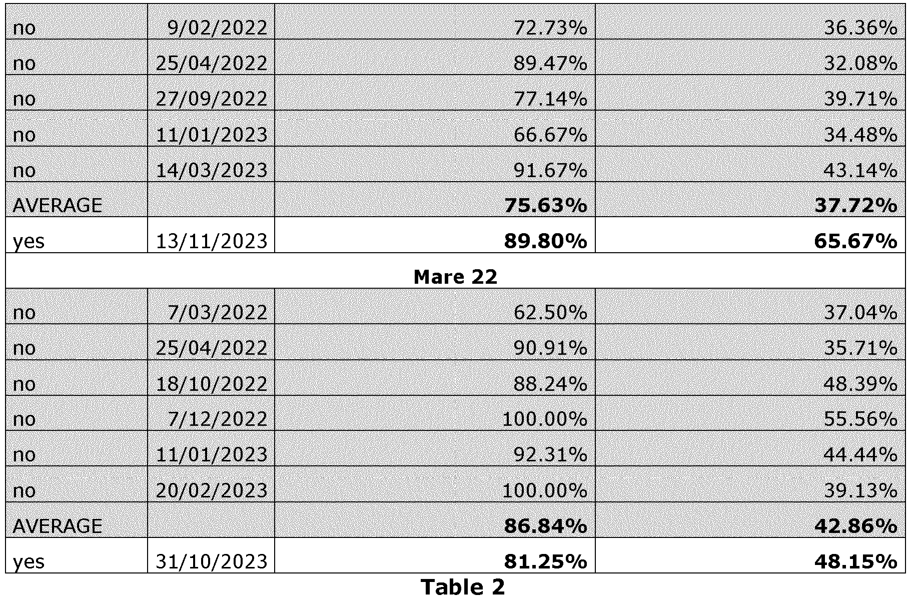

### CONCLUSION

The feed additive according to the current invention contributes to improved oocyte maturation, thereby increasing the number of follicles that can be punctured and the percentage of oocytes that have undergone successful division. Moreover, the number of fertilized oocytes that successfully develop into a blastocyst has increased. The current invention is capable of optimizing the methylation pattern in the oocyte, which is crucial for oocyte maturation and the successful development of the embryo. In the early stages of embryonic development, there is namely a need for epigenetic reprogramming, including extensive changes in DNA methylation patterns. Disrupted methylation can affect the integrity of the genome and epigenome, leading to genetic mutations or abnormal epigenetic patterns, with potentially adverse consequences for embryo development.

## Claims

1. A composition for use in treating ovulatory sub- or infertility in equids, the composition comprising a mixture of one or more methyl donors.

2. The composition for use according to claim 1, **characterized in that** the methyl donor is betaine and/or a precursor of betaine, such as choline and/or a choline derivative.

3. The composition for use according to claim 2, wherein the composition comprises between 5 and 15 weight percent choline or a choline derivative and between 8 and 12 weight percent betaine.

4. The composition for use according to any of the preceding claims, wherein the composition further comprises glucobrassicin or a source of glucobrassicin.

5. The composition for use according to any of the preceding claims, wherein the composition further comprises sulforaphane and/or one or more plant components as a source of sulforaphane.

6. The composition for use according to any of the preceding claims, wherein the composition further comprises lignans or a source of lignans.

7. The composition for use according to any of the preceding claims, wherein the composition further comprises cysteine.

8. The composition for use according to any of the preceding claims 5-7, **characterized in that** the composition contains broccoli sprouts as a source of glucobrassicin and/or sulforaphane, where the broccoli sprouts are present in the form of a broccoli extract containing at least 10 weight percent glucosinolates, based on the total weight of the broccoli extract.

9. The composition for use according to any of the preceding claims 6-8, **characterized in that** the composition contains linseed extract as a source of lignans, where the linseed extract contains at least 20 weight percent lignans, based on the total weight of the linseed extract.

10. The composition for use according to claim 9, the composition comprising:
- Between 15 and 25 weight percent broccoli extract;
- Between 5 and 23 weight percent linseed extract;
- Between 10 and 15 weight percent cysteine;
- Between 5 and 15 weight percent choline or a choline derivative;
- Between 8 and 12 weight percent betaine.

11. A method for collecting one or more unfertilized oocytes from the follicles of the ovaries of a subject of the equid group, wherein prior to collecting the one or more unfertilized oocytes, a composition is administered to the subject, the composition comprising a mixture of one or more methyl donors.

12. A method for ex *vivo* fertilizing one or more oocytes of a subject of the equid group, the method comprising:
- collecting one or more unfertilized oocytes from the subject;
- performing ICSI (Intracytoplasmic Sperm Injection) and/or IVF (*in vitro* fertilization) for fertilizing the one or more oocytes,
**characterized in that** prior to collecting the one or more unfertilized oocytes, a composition is administered to the subject, the composition comprising a mixture of one or more methyl donors.

13. The method according to any of the preceding claims 11-12, wherein the
composition further comprises glucobrassicin or a source of glucobrassicin, sulforaphane and/or one or more plant components as a source of sulforaphane, lignans or a source of lignans, and/or cysteine.

14. A feed additive for equids comprising:
- Between 15 and 25 weight percent broccoli extract;
- Between 5 and 23 weight percent linseed extract;
- Between 10 and 15 weight percent cysteine;
- Between 5 and 15 weight percent choline or choline derivative;
- Between 8 and 12 weight percent betaine.
